# EUROPEAN PATENT APPLICATION

(11) **EP 4 553 166 A1**
(43) Date of publication of application: **14.05.2025**
(21) Application number: 23208361.8
(22) Date of filing: 07.11.2023
(51) Int. Cl.: C12Q 1/18, A61K 38/50, C12N 9/86, G01N 33/569

(54) **METHOD FOR PREPARING A SAMPLE FOR ANALYSIS**

(71) Applicant: MinervaX ApS, 2000 Frederiksberg (DK)
(72) Inventor: BAHNAN, Wael, 226 52 Lund (SE); GONZALEZ-MIRO, Majela, 222 21 Lund (SE); JOHANSSON LINDBOM, Bengt, 224 57 Lund (SE)
(74) Representative: Brann AB

(57) **Abstract**

The technology proposed herein relates to a method for preparing a sample for analysis, said method comprising: a) providing a sample potentially containing an antimicrobial substance, such as an antibiotic; and b) treating said sample with an enzyme to inactivate said antimicrobial substance. A method comprising a microbial viability assay of a sample, a New Delhi metallo-beta-lactamase 1 (NDM-1) construct for recombinant expression thereof, a method for recombinantly producing a New Delhi metallo-beta-lactamase 1 (NDM-1) protein, and a use of an antimicrobial inactivating enzyme for preparing a sample for an analysis are also disclosed.

## Description

### FIELD OF INVENTION

The technology proposed herein relates to the fields of microbiology and vaccine technology and concerns a method for preparing a sample for analysis. More particularly, the technology proposed herein relates to a method for preparing a sample for analysis, such that any residual content of antimicrobial substances in the sample does not interfere with the later analysis.

### BACKGROUND OF THE INVENTION

Group B Streptococcus (*Streptococcus agalactiae*) (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease. GBS can also cause mastitis in cows, a bovine disease that is of considerable economic importance.

Development of a vaccine against GBS infections is therefore of interest.

An integral part of vaccine development is the testing of vaccine efficacy by analyzing samples from vaccinated subjects for determining the protective effect of a vaccine. Such samples may however contain further substances which may interfere with the analysis. Test subjects may for example be treated with antimicrobial substances, either for the specific microorganism the vaccine is to protect against, or for another microbe. In either case the substances may interfere with the analysis so that the corresponding samples must be discarded. This is disadvantageous both for the developer of the vaccine, due to the costs involved with enrolling and managing further test subjects in clinical studies, and for the test subject.

Accordingly, it is a primary objective of the technology proposed herein to provide a method for preparing a sample for analysis which method prevents and/or removes the negative effects of the potential presence of one or more antimicrobial substances in the sample.

It is a second objective of the technology proposed herein to provide a method comprising a microbial viability assay of a sample, in which method the negative effects of the potential presence of one or more antimicrobial substances in the sample is accounted for and/or at least party neutralized.

It is a third objective of the technology proposed herein to provide a New Delhi metallo-beta-lactamase 1 (NDM-1) polypeptide which inactivates antimicrobial substances.

It is at fourth object of the technology proposed herein to provide a method for recombinantly producing a New Delhi metallo-beta-lactamase 1 (NDM-1) protein.

It is further a fifth object of the technology proposed herein to provide a use of an antimicrobial inactivating enzyme for preparing a sample for an analysis.

It is further a sixth object of the technology proposed herein to provide a nucleic acid sequence encoding a New Delhi metallo-beta-lactamase 1 (NDM-1) protein or variant thereof.

It is further a seventh object of the technology proposed herein to provide a vector, such as a plasmid vector, comprising such a nucleic acid sequence.

It is an eight object of the technology proposed herein to provide a host cell comprising such a nucleic acid or vector.

### SUMMARY OF THE INVENTION

A first aspect of the technology proposed herein relates to a method for preparing a sample for analysis, wherein said method comprises:
a) providing a sample potentially containing an antimicrobial substance, such as an antibiotic, and
b) treating said sample with an enzyme to inactivate said antimicrobial substance.

The present invention is accordingly based on the discovery that otherwise unusable samples may still be used in the analysis provided that the activity of the antimicrobial substance contained in the sample is inactivated using the enzyme. As shown in the examples, this inactivation prevents killing of any microbes involved in the subsequent analysis. This allows the sample to be used in the analysis without the risk of incorrect analysis results.

A second aspect of the technology proposed herein relates to a method comprising a microbial viability assay of a sample, said method comprising the steps of:
a) preparing a sample for analysis according to the method of the first aspect; and
b) performing a microbial viability assay on the prepared sample of step a).

A third aspect of the technology proposed herein relates to a recombinant New Delhi metallo-beta-lactamase 1 (NDM-1) polypeptide, said NDM-1 polypeptide comprising an amino acid sequence according to SEQ ID NO: 2, or a variant thereof having at least 95% sequence identity thereto.

A fourth aspect the technology proposed herein relates to a method for recombinantly producing a New Delhi metallo-beta-lactamase 1 (NDM-1) protein according to SEQ ID NO: 1 or a variant thereof having at least 95% sequence identity thereto, said method comprising the steps of:
a) expressing a polypeptide according to SEQ ID NO: 2, or a variant thereof having at least 95% sequence identity thereto, in a bacterial host cell, such as an *E. coli* host cell, and allowing said bacterial host to cleave off a leader sequence from SEQ ID NO: 2, thereby providing an amino acid sequence according to SEQ ID NO: 3, or a variant thereof having at least 95% sequence identity thereto;
b) optionally lysing said bacterial host cell to release said amino acid sequence according to SEQ ID NO: 3, or said variant thereof, from the bacterial host cell;
c) using the His-tag of SEQ ID NO: 3, or the variant thereof, and affinity chromatography to obtain said amino acid sequence of SEQ ID NO: 3, or said variant thereof; and
d) using a TEV protease to cleave cleaving the His-tag off from the amino acid sequence of SEQ ID NO: 3, or said variant thereof, thereby rendering an NDM-1 protein according to SEQ ID NO: 1, or a variant thereof having at least 95% sequence identity thereto.

A fifth aspect of the technology proposed herein relates to the use of an antimicrobial inactivating enzyme as defined herein for preparing a sample for an analysis as defined herein.

Sixth, seventh, and eight aspects of the technology proposed herein relates to a nucleic acid sequence encoding an amino acid sequence of SEQ ID NO: 2 or encoding a variant of the amino acid sequence of SEQ ID NO: 2 having at least 95% sequence identity thereto, a vector, such as a plasmid vector, comprising such a nucleic acid, and a host cell comprising such a nucleic acid or vector, respectively.

Other features and advantages of the technology proposed herein will be apparent from the following detailed description, drawings, examples, and from the claims.

### BRIEF DESCRIPTION OF THE FIGURES

- Fig. 1: shows survival of GBS bacteria expressing the respective AlpC, Rib, Alp1, and Alp2 surface proteins in untreated and NDM-1 treated cord blood serum from infants born by mothers being treated with antibiotics during or close to delivery.
- Fig. 2: shows survival of GBS bacteria in human serum spiked with antibiotics and treated with successively higher concentrations of NDM-1
- Fig. 3A-B: show OPkA analysis results for a serum sample analyzed without supplementations, with addition of NDM-1 alone or in combination with various antibiotics.
- Fig. 4: shows OPkA analysis results for several sera treated with NDM-1 alone or in combination with various antibiotics.

### DETAILED DESCRIPTION OF THE INVENTION

As above, a first aspect of the present invention relates to a method for preparing a sample for analysis, wherein said method comprises:
a) providing a sample potentially containing an antimicrobial substance, such as an antibiotic, and
b) treating said sample with an enzyme to inactivate said antimicrobial substance.

In a second aspect, related to the first aspect, the technology proposed herein relates to a method comprising a microbial viability assay of a sample. The method comprising a microbial viability assay of a sample comprises the steps of a) preparing a sample for analysis according to the method of the first aspect and b) performing a microbial viability assay on the prepared sample of step a).

Accordingly, the present inventors have considered that, when performing an assay of a sample, it is very important that no other factors but the factor to be studied, e.g., whether or not a vaccine has generated an immune response, influences the result. This is particularly a problem when the analysis to be performed involves determining microbial viability and when the sample contains antimicrobial substance(s). Antimicrobial substances in a sample inevitably will risk interfering with any assay involving microbial viability or microbial killing measurement and skew the results. However, due to the widespread use of antimicrobial substances, such substances are present in many samples, sometimes without the subject or the treating medical doctor being aware of it. This leads to problems when performing assays involving microbial viability measurements as it has not been possible to trust the results without knowledge of the antimicrobial treatment history of the subject.

However, as set out in method according to the first aspect of the technology proposed herein, the present inventors surprisingly found that by employing an enzyme inactivating antimicrobial substances in a pre-treatment step before analysis of a sample, it was possible to dispense with the need for information regarding any antimicrobial treatment of the subject. Consequently, samples can be used without the need for the subject to be free from antimicrobial substances. This represents a major advantage since samples neither need to be tested for antimicrobial substances, nor discarded if antimicrobial substances are found. Further, there is no longer a need to remove subjects from a clinical study for vaccine efficacy even if/when the subjects are treated with antimicrobial substances. Correspondingly, there is no need to overpopulate a clinical study to compensate for unusable samples due to treatment with antimicrobial substances.

The method for preparing a sample for analysis may alternatively be considered a method for pretreating a sample for analysis, a method for conditioning a sample for analysis, and/or a method for reducing the risk that substances in a sample interfere with a subsequent analysis. In particular, the method may be considered a method of purifying a sample by removing or inactivating one or more substances in the sample. The methods according to the first and second aspects of the technology proposed herein do not constitute treatment of the subject, nor do they constitute diagnostic methods. Rather, the methods provide for preparing a sample for subsequent analysis and a method comprising a microbial viability assay of a sample.

Preparing encompasses treating, pretreating, and conditioning. Preparing may further comprise purifying and/or removing an interfering substance from the serum sample.

The sample is preferably a biological sample. The sample may be a liquid sample. Preferably the sample is selected from the group consisting of whole blood, plasma, serum, saliva, and urine. The sample may also be a solid sample such as faeces or a tissue sample. If the sample is a solid sample, then the sample may be homogenized and/or otherwise disintegrated before being treated with the enzyme.

Preferably the sample is a serum sample. Serum is the clear, yellowish fluid remaining after clotting factors (such as fibrinogen and prothrombin) have been removed from blood plasma by clot formation. The serum sample may be obtained from a subject, such as human or animal subject. In particular, the serum sample may be a serum sample from a vaccinated subject, such as a vaccinated female subject. Further, the serum sample may be obtained from the offspring of a vaccinated female subject.

The serum sample may be a serum sample from a subject that is or has been treated with one or more antimicrobial substance(s) or the offspring of such a subject. As mentioned elsewhere herein, by treating the serum sample of a subject according to the first and second aspect of the technology proposed herein, it is ensured that antimicrobial substance(s) potentially present in the serum sample is inactivated before further analysis of the serum sample.

"For analysis" encompasses that the sample is prepared to render it suitable for a subsequent analysis that is performed on the sample after the sample has been prepared using the method according to the first aspect of the technology proposed herein.

The sample may be provided by way of for example a standard blood test. Providing the sample does not involve treatment of the human or animal body by therapy or surgery. In particular, blood tests and/or blood samples obtained as routine for other uses may be used to provide the sample for the methods according to the first and second aspects of the technology proposed herein.

The sample may be from a subject. The subject may be a human or animal subject. In particular, the subject may be a vaccinated subject, e.g., a subject vaccinated with a vaccine against a microbe. Typically, the subject may be a female subject, e.g., a vaccinated female subject, or the offspring of such a female subject.

The methods according to the first and second aspects of the technology proposed herein refer to a sample potentially containing an antimicrobial substance. The term potentially thus encompasses that it is not known whether the sample contains the antimicrobial substance, that it is expected that the sample contains the antimicrobial substance, that it is known that the sample contains the antimicrobial substance, and that it has been determined, by an analysis on the sample which detects the antimicrobial substance, that the sample contains the antimicrobial substance. Preferably potentially means that the sample contains the antimicrobial substance.

The sample may in particular be from a subject potentially treated with the antimicrobial substance. Alternatively, the sample is from the offspring of a subject potentially treated with the antimicrobial substance. Potentially treated has the same corresponding possible meanings as given for potentially containing above, mutatis mutandis.

Being treated with an antimicrobial substance comprises that the sample contains the antimicrobial substance. Preferably treated with an antimicrobial substance comprises that the sample contains the antimicrobial substance in active form, i.e., the antimicrobial substance is present in the sample in a form, e.g., its normal, functional form, in which it exerts and antimicrobial effect on microbes.

Analogously, the sample containing the antimicrobial substance preferably corresponds to the sample containing the antimicrobial substance in a form, e.g., its normal, functional form, in which it exerts and antimicrobial effect on microbes.

The antimicrobial substance is any antimicrobial substance that exerts an antimicrobial effect. The antimicrobial substance is further understood to encompass antimicrobial substances that can be enzymatically inactivated. Such enzymatic inactivation may e.g., involve hydrolysis, transfer of functional groups or redox reactions.

Typically, the antimicrobial substance is an antibiotic. An antibiotic is any substance that kills bacteria.

"Treating" is to be understood as encompassing contacting as well as mixing. Thus, treating the sample with the enzyme may comprise any of mixing the sample with the enzyme, where the enzyme may be free or immobilized on a carrier, passing the sample along a surface on which the enzyme is immobilized, etc.

The sample is treated with the inactivating enzyme for a time sufficient to inactivate all or substantially all of any antimicrobial substance(s) in the sample. By "substantially all" is in the context of the technology proposed herein intended that enough of any antimicrobial substance(s) in the sample are inactivated to not interfere with a later analysis of the sample. Typically, a sufficient time is a time whereafter the sample does not affect the viability of microbes subjected to the sample.

The enzyme inactivating the antimicrobial substance may be any enzyme that can inactivate an antimicrobial substance. Such enzymes may in context of the technology proposed herein be denoted "inactivating enzyme" and the like.

By "inactivate" and the like as used herein is intended any treatment that results in an inactive antimicrobial substance, i.e., an antimicrobial substance that no longer exerts an antimicrobial effect, preferably its intended antimicrobial effect, on microbes. Such inactivation may for example involve, but is not limited to, a cleavage (hydrolysis) of the antimicrobial molecule (such as by hydrolases) or a formation of inactive derivatives (such as by transferases or redox enzymes) of the antimicrobial molecule. Preferably, the inactivation comprises a cleavage (hydrolysis) of the antimicrobial molecule (such as by hydrolases).

Performing a microbial viability assay comprises performing an assay in which the viability of microbes is determined, and in particular an assay in which the viability of microbes is used as an indication for determining a property of the sample.

In the context of the technology proposed herein a prepared sample is a sample which has been treated with an enzyme to inactivate an antimicrobial substance in the sample.

As mentioned above, when samples from a subject that are or have been treated with an antimicrobial substance are to be analysed, there is a risk that the presence of antimicrobial substance(s) in the sample interferes with the analysis. Therefore, removing or inactivating the antimicrobial substance may be of outmost importance in order for the analysis to provide a reliable result unless it can be ensured that the sample is free from antimicrobial substances, which rarely is the case.

The problem with presence of antimicrobial substance(s) in a sample is in particular a problem when the analysis comprises determining microbial viability, preferably wherein said analysis comprises determining an immunological activity against microbes. Determining microbial viability may comprise measuring or studying microbial viability. In particular an analysis comprising determining microbial viability may use the viability of a microbe, e.g., whether a microbe or population of microbes survives, multiplies, goes dormant, or dies, as an indication for determining a property of the sample. The microbes may be fungi or bacteria, preferably bacteria. The analysis may thus comprise exposing a microbe or population of microbes to the sample, alone or in combination with other compounds, to determine whether the sample has or elicits an immunological activity against the microbe.

Preferably, the analysis is an OpsonoPhagocytosis killing Assay (OPkA assay). Generally, opsonophagocytosis is the engulfment, by macrophages and other phagocytic cells like neutrophils, of bacteria opsonized with antibodies and/or complement proteins. Thus, microbial cells in the blood or within a specific tissue that become coated with IgG are soon recognized by patrolling macrophages and neutrophils. Typically, recognition occurs through specific surface-expressed FcyRs on the phagocytes, but the effect may also involve complement receptors. When multiple FcγRs are engaged simultaneously, it cues the phagocyte to envelope its prey and confine it within an intracellular compartment known as the phagosome. The phagosome is a dead-end for most microbial pathogens. Within minutes, the encased bacteria are exposed to reactive oxygen species, acidic pH and a barrage of degradative enzymes.

In an OPkA assay, phagocytic cells are co-cultured with bacterial cells. The phagocytic immune cells will phagocytose and kill the bacterial cells in a complement-dependent manner and the assay can be used to study the ability of such phagocytic immune cells to respond to and kill bacterial cells under different conditions. In particular, the OpkA assay can be used to measure the functionality of antibodies and for example assess protective immunity of Group B Streptococcus (GBS) vaccines. The efficiency of vaccines can thus be studied using an OpkA assay. In particular, the OpkA analysis can determine whether vaccination with a vaccine, e.g., a GBS vaccine candidate, results in the vaccinated subjects obtaining opsonizing antibodies, i.e., antibodies which when contacted with bacteria, bind to the bacteria and induce phagocytic cells to phagocytose the bacteria. The OPkA assay is preferably configured for assaying the binding of antibodies to GBS bacteria, in particular to surface proteins of GBS bacteria. Such an OPkA assay preferably involves the binding of IgG to the GBS bacteria leading to a cascade of complement activation, resulting in deposition of C3b on the bacterial surface. This leads to phagocytosis by neutrophils through recognition of C3b by complement receptors on the neutrophils.

Preferably, the antibiotic is a beta-lactam antibiotic. Beta-lactam antibiotics are so called "broad spectrum" antibiotics and are among the most widely used group of antibiotics. The antibiotic to be inactivated according to the technology proposed herein may thus for example be a beta-lactam. Beta-lactams are mainly active against Gram-positive bacteria, although beta-lactam antibiotics active against Gram-negative bacteria have also been developed.

Preferably the beta-lactam antibiotic is a penicillin, Cephalosporin, cephamycin, monobactam, carbapenem or carbacephems.

More preferably, the beta lactam antibiotic is penicillin, cephalosporin or carbapenem.

The enzyme is preferably a beta-lactamase, preferably a carbapenemase, more preferably a class B carbapenemase. Beta-lactamases are a group of enzymes that provide bacterial resistance to beta-lactam antibiotics by breaking the antibiotic's structure by hydrolysis. The beta-lactamases used according to the technology proposed herein are preferably recombinantly produced. Carbapenemases are members of the molecular class A, B, or D β-lactamases. Class A and D enzymes have a serine-based hydrolytic mechanism, while class B enzymes are metallo-β-lactamases containing zinc in their active site. Carbapenemases can inactivate not only carbapenems but e.g also other broad-spectrum antibiotics such as penicillins, oxymino-cephalosporins, and cephamycins and are thus suitable to use as the inactivating enzyme according to the technology proposed herein as they can inactivate a wide range of antibiotics.

The carbapenemase is preferably a class B carbapenemase.

The class B carbapenemase may preferably be a New Delhi metallo-beta-lactamase (NDM). There are several variants of NDM, with NDM-1 being the most spread across different hosts. The variants have small genetic differences across them ranging between 1 to 5 amino acid substitutions compared to NDM-1 (maximum % variation is 5/270 amino acids)

Even more preferably, the enzyme may be New Delhi metallo-beta-lactamase 1 (NDM-1). The NDM-1 enzymes confer resistance to all β-lactams except aztreonam and are therefore an attractive choice as the inactivating enzyme according to the technology proposed herein as it will inactivate the majority of beta-lactam antibiotics by cleaving the beta-lactam ring of such antibiotics.

Preferably, the NDM-1 used according to the technology proposed herein comprises are consists of an amino acid sequence according to SEQ ID NO: 1, or a variant thereof having at least 95% sequence identity thereto. This sequence is based on sequence NCBI ID: NG_049326.1 from *Klebsiella pneumoniae.*

Typically, the sample is treated with the inactivating enzyme for from about 10 minutes to about 40 minutes. The concentration of the inactivating enzyme when used in a method according to the technology proposed herein has to be adjusted depending on the specific enzyme used. Typically, nanomolar to micromolar concentrations of the enzyme is used, such as from about 10 nM to about 1 µM), such as from about 100 nM to about 500 nM, such as from about 100 nM to about 300 nM, such as about 200 nM (0.2 µM), in particular when NDM-1 is used as the inactivating enzyme.

Preferably, the sample is from a subject from that has been vaccinated against a bacterial infection, or is from the offspring of a subject that has been vaccinated against a bacterial infection. The subject is preferably a female subject.

In particular bacterial infection is a Group B streptococcal infection. Such a sample may contain antibodies against Group B streptococci. Such antibodies may be studied using the above referred to OPkA assay.

The method according to the first aspect of the technology proposed herein may comprise the step of performing a microbial viability assay on the prepared sample, i.e., as per step (b) of the method according to the second aspect of the technology proposed herein.

Preferably, the subject from which the sample is provided has been vaccinated, or is the offspring of a female subject who has been vaccinated, against Group B streptococcal (GBS) infection using the immunogenic fusion protein disclosed in WO2008/127179 or WO 2022/207657, alone or in combination with the immunogenic fusion protein disclosed in WO2017/068112.

More particularly, the subject from which the sample is provided has been vaccinated, or is the offspring of a female subject who has been vaccinated, against Group B streptococcal (GBS) infection using a combination of an immunogenic fusion protein comprising the N terminals of the Rib and AlpC GBS surface proteins, combined with a further immunogenic fusion protein comprising the N terminals of the Alp1 and Alp2 GBS surface proteins.

More preferably, the subject from which the sample is provided has been vaccinated, or is the offspring of a female subject who has been vaccinated, against Group B streptococcal (GBS) infection using a GBS vaccine candidate comprises an immunogenic fusion protein consisting of SEQ ID NO: 5 combined with a further immunogenic fusion protein consisting of SEQ ID NO: 6 in a buffer further comprising the adjuvant aluminum hydroxide. In each of these cases the sample is preferably a serum sample.

The third aspect of the technology proposed herein is related to a New Delhi metallo-beta-lactamase 1 (NDM-1) polypeptide wherein the polypeptide comprises or consists of an amino acid sequence according to SEQ ID NO: 2, or a variant thereof having at least 95% sequence identity thereto. SEQ ID NO: 2 comprises an amino acid sequence for an NDM-1 protein, such as the NDM-1 protein of SEQ ID NO: 1 or a variant thereof as described elsewhere herein, preceded by a PelB leader sequence, a His-tag and a TEV protease recognition site on the N-terminal side of the NDM-1 amino acid sequence. The PelB leader sequence has the function of ensuring that the produced peptide is directed to the bacterial periplasm. Once in the periplasm, the PelB leader sequence is removed by a signal peptidase. The function of the His-tag is to allow the protein to be purified by affinity chromatography. The function of the TEV protease recognition site is to allow the His-tag to be cleaved off, rendering the final NDM-1 protein according to SEQ ID NO: 3. This polypeptide is therefore optimized for recombinant expression and purification of an NDM-1 protein.

The fourth aspect the technology proposed herein relates to a method for recombinantly producing a New Delhi metallo-beta-lactamase 1 (NDM-1) protein according to SEQ ID NO: 1 or a variant thereof having at least 95% sequence identity thereto, said method comprising the steps of:
a) expressing a polypeptide according to SEQ ID NO: 2, or a variant thereof having at least 95% sequence identity thereto, in a bacterial host cell, such as an *E. coli* host cell, and allowing said bacterial host to cleave off a leader sequence from SEQ ID NO: 2, thereby providing an amino acid sequence according to SEQ ID NO: 3, or a variant thereof having at least 95% sequence identity thereto;
b) optionally lysing said bacterial host cell to release said amino acid sequence according to SEQ ID NO: 3, or said variant thereof, from the bacterial host cell;
c) using the His-tag of SEQ ID NO: 3, or the variant thereof, and affinity chromatography to obtain said amino acid sequence of SEQ ID NO: 3, or said variant thereof; and
d) using a TEV protease to cleave the His-tag off from the amino acid sequence of SEQ ID NO: 3, or said variant thereof, thereby rendering an NDM-1 protein according to SEQ ID NO: 1, or a variant thereof having at least 95% sequence identity thereto.

Expressing comprises that a DNA sequence corresponding to the amino acid sequence according to SEQ ID NO: 2, or the variant thereof having at least 95% sequence identity thereto, is inserted, transiently or stably, into the bacterial host cell so that the bacterial host cell produces, i.e., expresses, the amino acid sequence according to SEQ ID NO: 2, or the variant thereof having at least 95% sequence identity thereto.

The bacterial host cell should be able to express a signal peptidase that can cleave the PelB leader sequence off from the expressed amino acid sequence of SEQ ID NO: 2, or the variant thereof, resulting in an amino acid sequence according to SEQ ID NO: 3.

The bacterial host cell is typically an *E. coli* host cell since these types of host cells are commonly used and easy to utilize.

Generally, no special step or measures are needed for allowing said bacterial host to cleave off the leader sequence, as long as the bacterial host cell has the capacity, i.e., has within it, the proteins and/or enzymes needed to cleave of the leader sequence. Accordingly, no special step or measures are generally needed for providing the amino acid sequence according to SEQ ID NO: 3 or the variant thereof having at least 95% sequence identity thereto.

In optional step b) of the method according to the fourth aspect, the bacterial host cells that have produced an amino acid sequence according to SEQ ID NO: 3, or said variant thereof, are lysed, i.e., treated to obtain said amino acid sequence in solution. Such lysing involves disrupting the bacterial host cell so that the intracellular material, including the amino acid sequence according to SEQ ID NO: 3, or said variant thereof, is released into the solution in which the bacterial host cell is grown or stored. Techniques for cell lysis are well-known to the person skilled in the art and include, but are not limited to, sonication, freeze-thawing, manual grinding, osmotic shock, enzymatical lysis, use of detergents etc. The cell lysis results in the amino acid sequence according to SEQ ID NO: 3, or said variant thereof, to become accessible for affinity chromatography purification thereof using the His-tag expressed.

Although step (b) is optional, it is preferred. If step b is not performed, then an expression system configured to cause the amino acid sequence according to SEQ ID NO: 3, or said variant thereof, to be transported out of the bacterial host cell into the solution in which the bacterial host cell is grown or stored, may be used instead.

In step c) the amino acid sequence according to SEQ ID NO: 3, or said variant thereof, is purified by using affinity chromatography by using a chromatography matrix binding to the His-tag of the amino acid sequence according to SEQ ID NO: 3, or said variant thereof. This allows the amino acid sequence according to SEQ ID NO: 3 to be obtained, from the solution in which the bacterial host cell was grown or stored as the solution is passed through an affinity column, i.e., a column in which the solid phase carries ligands having affinity for the His tag. This allows the amino acid sequence to be separated from the solution.

It is further contemplated within the context of the technology proposed herein that antibody mediated purification can be used as an alternative to affinity chromatography in step (c) to separate said amino acid sequence of SEQ ID NO: 3, or said variant thereof, from said solution of step b).

This His-tag is typically cleaved off using a TEV protease to render, e.gg. provide or obtain, the NDM-1 protein according to SEQ ID NO: 1, or a variant thereof having at least 95% sequence identity thereto.

The method according to the fourth aspect may also comprise a step of separating the NDM-1 protein from the cleaved off His-tag and the TEV protease, such as by affinity chromatography and size exclusion chromatography.

A fifth aspect the technology proposed herein relates to the use of an antimicrobial inactivating enzyme as defined herein for preparing a sample for an analysis as defined elsewhere herein.

Sixth, seventh, and eight aspects of the technology proposed herein relates to a nucleic acid sequence encoding an amino acid sequence of SEQ ID NO: 2 or encoding a variant of the amino acid sequence of SEQ ID NO: 2 having at least 95% sequence identity thereto, a vector, such as a plasmid vector, comprising such a nucleic acid, and a host cell comprising such a nucleic acid or vector, respectively.

Whenever an amino acid sequence is referred to herein, this also includes a variant of said amino acid sequence, wherein said variant has a sequence identity of at least 95%, such as at least 96%, such as at least 97%, such as at least 98%, such as at least 99% to the sequence referred to. According to the technology proposed herein, sequence identity is calculated in the following manner. By an amino acid sequence having a sequence at least, for example 95%, identity to a reference sequence, is intended that the sequence is identical to the reference sequence except that the sequence may include up to 5 amino acid differences per each 100 amino acids of the reference sequence. In other words, to obtain an amino acid sequence having a sequence identity of at least 95%, up to 5% of the amino acids in the reference sequence may be deleted or substituted with another amino acid, or a number of amino acids up to 5% of the total number of amino acids in the reference sequence may be inserted into the reference sequence. If the two sequences to be compared are not of equal length, they must be aligned to best possible fit, and the sequence identity calculated over the length of the reference sequence. These amino acid changes of the reference sequence may occur at the amino and/or carboxy terminal positions of a reference amino acid sequence or anywhere between those terminal positions, interspersed either individually within the reference sequence or in one or more contiguous groups within the reference sequence. Preferably, no substitutions, deletions or additions are present in the parts of the sequences referred to herein corresponding to the PelB leader sequence, the His-tag and/or the TEV protease recognition site.

Table 1 below details the sequences mentioned herein:

**Table 1: sequences**

| **SEQ ID NO** | **Sequence** | **Description** |
|---|---|---|
| SEQ ID NO: 1 | GQQMETGDQRFGDLVFRQLAPNVWQHTSY LDMPGFGAVASNGLIVRDGGRVLVVDTAWTD DQTAQILNWIKQEINLPVALAVVTHAHQDKMG GMDALHAAGIATYANALSNQLAPQEGMVAAQ HSLTFAANGWVEPATAPNFGPLKVFYPGPGH TSDNITVGIDGTDIAFGGCLIKDSKAKSLGNL GDADTEHYAASARAFGAAFPKASMIVMSHSA PDSRAAITHTARMADKLR | Amino acid sequence of NDM-1 protein |
| SEQ ID NO: 2 | MKYLLPTAAAGLLLLAAQPAMAMAAHHHHHH SSGVDLGTENLYFQGQQMETGDQRFGDLVF RQLAPNVWQHTSYLDMPGFGAVASNGLIVR DGGRVLVVDTAWTDDQTAQILNWIKQEINLPV ALAVVTHAHQDKMGGMDALHAAGIATYANAL SNQLAPQEGMVAAQHSLTFAANGWVEPATA PNFGPLKVFYPGPGHTSDNITVGIDGTDIAFG GCLIKDSKAKSLGNLGDADTEHYAASARAFG AAFPKASMIVMSHSAPDSRAAITHTARMADK LR | Recombinantly expressed NDM-1 construct including a PelB leader sequence, a His-tag and a TEV protease recognition site |
| SEQ ID NO: 3 | HHHHHHSSGVDLGTENLYFQGQQMETGDQ RFGDLVFRQLAPNVWQHTSYLDMPGFGAVA SNGLIVRDGGRVLVVDTAWTDDQTAQILNWI KQEINLPVALAVVTHAHQDKMGGMDALHAA GIATYANALSNQLAPQEGMVAAQHSLTFAAN GWVEPATAPNFGPLKVFYPGPGHTSDNITVG IDGTDIAFGGCLIKDSKAKSLGNLGDADTEHY AASARAFGAAFPKASMIVMSHSAPDSRAAIT HTARMADKLR | Corresponds to SEQ ID NO: 2 but with the PelB leader sequence cleaved off |
| SEQ ID NO: 4 | MELPNIMHPVAKLSTALAAALMLSGCMPGEIR PTIGQQMETGDQRFGDLVFRQLAPNVWQHT SYLDMPGFGAVASNGLIVRDGGRVLVVDTAW TDDQTAQILNWIKQEINLPVALAVVTHAHQDK MGGMDALHAAGIATYANALSNQLAPQEGMV AAQHSLTFAANGWVEPATAPNFGPLKVFYPG PGHTSDNITVGIDGTDIAFGGCLIKDSKAKSL GNLGDADTEHYAASARAFGAAFPKASMIVMS | NDM-1 from *Klebsiella* NCBI ID: NG_049326.1 |
| | HSAPDSRAAITHTARMADKLR | |
| SEQ ID NO: 5 | MAEVISGSAVTLNTNMTKNVQNGRAYIDLYD VKNGKIDPLQLITLNSPDLKAQYVIRQGGNYF TQPSELTTVGAASINYTVLKTDGSPHTKPDG QVDIINVSLTIYNSSALRDKIDEVKKKAEDPKW DEGSRDKVLISLDDIKTDIDNNPKTQSDIANKI TEVTNLEKILVPRIPEFSTIPGSAATLNTSITKNI QNGNAYIDLYDVKLGKIDPLQLIVLEQGFTAKY VFRQGTKYYGDVSQLQSTGRASLTYNIFGED GLPHVKTDGQIDIVSVALTIYDSTTLRDKIEEV RTNANDPKWTEESRTEVLTGLDTIKTDIDNNP KTQTDIDSKIVEVNELEKLLVLS | The sequence of the first immunogenic fusion protein used in the GBS vaccine candidate in Example 1 comprising the N-terminals or Rib and AlpC |
| SEQ ID NO: 6 | MAEVISGSAATLNSALVKNVSGGKAYIDIYDV KNGKIDPLNLIVLTPSNYSANYYIKQGGRIFTS VNQLQTPGTATITYNILDENGNPYTKSDGQIDI VSLVTTVYDTTELRNNINKVIENANDPKWSDD SRKDVLSKIEVIKNDIDNNPKTQSDIDNKIVEV NELEKLLVLPEFSTIPGSAATLNTSITKNIQNG NAYIDLYDVKNGLIDPQNLIVLNPSSYSANYYI KQGAKYYSNPSEITTTGSATITFNILDETGNP HKKADGQIDIVSVNLTIYDSTALRNRIDEVINN ANDPKWSDGSRDEVLTGLEKIKKDIDNNPKT QIDIDNKINEVNEIEKLLVVSL | The sequence of the second immunogenic fusion protein used in the GBS vaccine candidate in Example 1 comprising the N-terminals or Alp1 and Alp2 |

The technology proposed herein will be further described in the following examples, which do not limit the scope of the invention described in the claims.

Although the technology proposed herein will be illustrated in the experimental section in connection with a OPkA assay, the technology proposed herein according to the different aspects can of course be used also in connection with other assays where remaining antimicrobials may interfere with the assay as disclosed elsewhere herein. Thus, the technology proposed herein can be used in connection with any assay where it is important that any remaining antimicrobials do not interfere with the assay. As mentioned elsewhere herein, the technology proposed herein is particularly suitable to use in assays involving microbial viability such as for example Complement deposition assays, such as Crb deposition assays, and bactericidal assays

Moreover, although the serum sample analysed in Example 1 is a serum sample from subject vaccinated with a GBS vaccine candidate, the technology proposed herein is not limited to assays of vaccination efficiency, nor to serum samples, but is suitable for analysis of any type of biological samples where antimicrobial presence may interfere with the subsequent analysis.

### Example 1 - Survival of GBS in maternal antibiotic-containing serum samples in the absence or presence of NDM-1.

In this example, various serum samples were treated with a recombinant NDM-1 enzyme according to the third aspect of the technology proposed herein to ascertain whether the treatment of the serum samples would inactivate antimicrobial substances, specifically the antibiotics listed in table 2 below, sufficiently so that GBS bacteria could survive in the serum samples and the serum samples thus could be subjected to an OPkA assay without the risk of incorrect results.

**Table 2: Antibiotics found in serum samples**

| **Antibiotic found in serum sample** | **Total number of subjects/serum samples** | **Number of Sera with remaining antibiotic activity** | **% of Sera with remaining antibiotic activity** |
|---|---|---|---|
| Ampicilin | 3 | 1 | 33 |
| Ampiclox | 8 | 2 | 25 |
| Cefazolin | 25 | 9 | 36 |
| Kefzol | 16 | 11 | 69 |
| Ceftriaxone | 19 | 11 | 58 |
| Augmentin | 6 | 3 | 50 |
| Cefotaxime | 1 | 1 | 100 |
| Cefixime | 1 | 0 | 0 |
| Ampicillin/Flagyl/Kefzol | 1 | 1 | 100 |
| Ampicillin/Gentamycin | 1 | 1 | 100 |
| Ampicillin/Cefazolin | 1 | 1 | 100 |
| Augmentin/Kefzol | 1 | 1 | 100 |
| (Azithromycin+Kefzol) | 1 | 0 | 0 |
| Total | 84 | 42 | 50 |

The antibiotics shown in table 2 all belong to the Penicillin or Cephalosporin groups. These are all beta lactam antibiotics and would thus be targets for the NDM-1 enzyme.

The serum samples in table 2 are infant cordblood serum samples from infants born to mothers who received antibiotics during delivery. These 42 subjects/samples represent about 50% of the number of subjects in a study into the efficacy of a GBS vaccine candidate. In the study, adult healthy women were immunized with the GBS vaccine candidate, which candidate comprised an immunogenic fusion protein consisting of SEQ ID NO: 5 combined with a further immunogenic fusion protein consisting of SEQ ID NO: 6 in a buffer further comprising the adjuvant aluminum hydroxide.

As the serum samples were intended to be further analyzed using a OPkA-analysis in order to determine whether the serum samples contained opsonizing antibodies, i.e., protective antibodies, there was a concern that the remaining antibiotic activity in the 42 serum samples would yield incorrect results. Specifically, it was a concern that remaining antibiotic activity in these serum samples could kill and otherwise affect the viability of GBS bacteria used in the OPkA analysis before the phagocytic cells of the analysis would have engulfed and killed those GBS bacteria as a result of antibodies in the serum samples binding to the GBS bacteria. In other words, a premature killing of the GBS bacteria used in the OPkA would cause the OPkA to incorrectly indicate that the serum samples concerned did in fact contain opsonizing antibodies, i.e., protective antibodies, against the GBS bacteria and that the GBS vaccine candidate thus was effective.

It was further noted that only 4% of the women in the study that received antibioitics during or close to delivery, had received antibiotics not being beta-lactams (e.g., Gentamycin). This indicated that a beta-lactamase could potentially inactivate all beta-lactams that could interfere with OPkA analysis in about 96& of all antibiotic-containing serum samples.

Accordingly, it was decided to try and see if the NDM-1 enzyme could inactivate the concerned antibiotics so that the 42 serum samples could be analyzed in the OPkA without this risk of incorrect results.

To this end, the serum samples were screened for any present GBS, and once it was found that no live GBS bacteria was found in the samples, 20 µL of each serum was incubated with or without 0.2 µM NDM-1 enzyme evaluate whether NDM-1 could cleave and inactivate the remaining beta-lactams. The incubation time was 30 minutes. After the incubation, 10 µL of titrated GBS stocks, i.e., live GBS bacteria, were added to the treated and untreated sera and incubated for an additional 30 minutes. The bacteria were then plated, and their viability assessed.

To ensure complete inactivation of the antibiotics against all GBS strains targeted by the GBS vaccine candidate, i.e., GBS serotypes expression the surface proteins Rib, AlpC, Alp1, and Alp2/3, all sera were tested against these GBS serotypes.

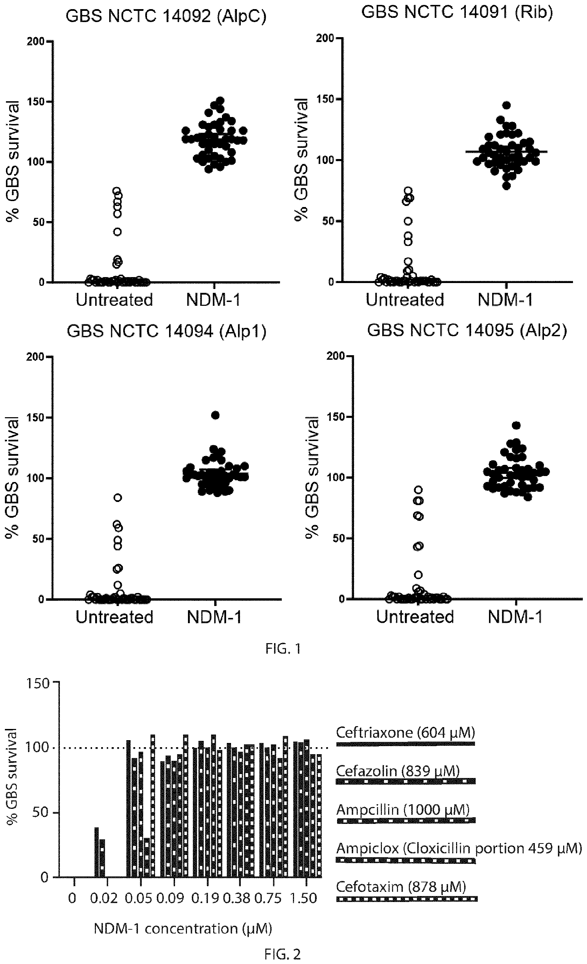

As seen in Fig 1, treatment of the serum samples with NDM-1 provided that the each of the four serotypes of GBS bacteria, i.e., GBS bacteria expressing each of the Rib, AlpC, Alp1 and Alp2/3 survived in the sera despite the initial content of the antibiotics. The treatment with NDM-1 has inactivated each of the antibiotics listed in table 2 such that the GBS bacteria can survive in the sera, wherefrom follows that these sera can now be subjected to the OPkA analysis to determine if the sera contained opsonizing antibodies. In other words, the use of NDM-1 in preparing serum samples that contain antibiotics allows the subsequent performance of an OPkA analysis which would otherwise be hampered by the antibiotics killing the bacteria in the background. While other beta lactamases could be used, none of them have as wide spectrum as NDM-1.

Not only did the use of the NDM-1 enzyme provide for using serum samples from infants born to mother who received antibiotics close to or during delivery, but many sera from IGbsD cases are likely to also contain antibiotics, which could prevent development of a CoP (correlate of protection) assay based on the OPkA. As beta-lactams are the preferred choice of antibiotics for prevention of IGbD, a beta-lactamase that efficiently inactivates all beta-lactams should also facilitate the development of a functional CoP.

### Example 2 - NDM-1 is effective in inactivating all clinically relevant concentrations of antibiotics at reasonable enzyme concentration

This example sought to elucidate the NDM-1 concentrations needed in, and thus the amount of NDM-1 to be added to, serum samples to efficiently inactivate all clinically relevant concentrations of antibiotics.

Accordingly, the survival of GBS was evaluated in normal human serum spiked with excessive concentrations of beta-lactams, in the absence or presence of graded doses of NDM-1. Normal human serum was thus spiked with high concentrations of beta-lactams [Ceftriaxone, Cefazolin (same as Kefzol), Ampiclox, Ampicillin and Cefotaxime].

Subsequently, the ability of graded NDM-1 concentrations to prevent killing of GBS when mixed with the spiked sera was evaluated. The only beta-lactam from table 2 not assessed in this example is Augmentin (a combination of the beta-lactam Amoxicillin and the betalactamase inhibitor clavulanic acid), which however is covered in the Example 3 further below. The concentration of antibiotics used corresponds to the maximal dose administered in the study of Example 1 divided by 5 liters; roughly corresponding to the total blood volume of an adult woman. By this approach, the concentration of beta-lactams in the spiked sera should by far exceed the maximal physiological serum concentrations reached following iv administration.

The example was conducted to mimic the initial step of the standard OPkA analysis; mixing 20 µL of serum with 10 µL of a titrated GBS stock (GASTON strain NCTC14091; Rib/CPS III) followed by an incubation at 37°C for 30 min. Of note, in OPkA analysis, undiluted serum is never used but instead 20 µL of serially diluted serum, starting at a 1:3 dilution, is added to the bacteria. After incubation, the mixture was plated and the CFU counted the day after. As shown in Figure 2, NDM-1 already at a concentration of 0.02 µM starts to promote bacterial survival and at a concentration of 0.19 µM (corresponding to 4.7 µg/mL) the enzyme has completely inhibited all five beta-lactams tested.

Accordingly, reasonable low concentrations of NDM-1 are capable of inactivating all clinically and physiologically relevant concentrations of the tested beta lactam antibiotics, thus providing for general use of the NDM-1 enzyme and the methods according to the first and second aspects of the technology proposed herein.

### Example 3 - NDM-1 does not interfere with an OPkA analysis

In this example it was tested whether the addition of NDM-1 to a serum sample would interfere with the performance of an OPkA analysis. The example further tested high concentrations of various beta lactams.

In the example, the NDM-1 enzyme was tested directly in OPkA analyses against all four GASTON GBS Alp serotypes. To this end, various sera (not containing antibiotics) were selected to cover a wide range of OPkA titers against all GBS target strains. Each of these sera has then been assayed (i) without any additions, (ii) with the addition of NDM-1, (iii) with the addition of NDM-1 plus a high concentration of one of in total six tested beta-lactams (Cefotaxime, Augmentin, Ampiclox, Cefazolin, Ceftriaxone and Ampicillin tested separately) and (iv) with NDM-1 plus a cocktail of three beta-lactams (fixed for all experiments and containing Ampicillin, Ceftriaxone and Cefazolin).

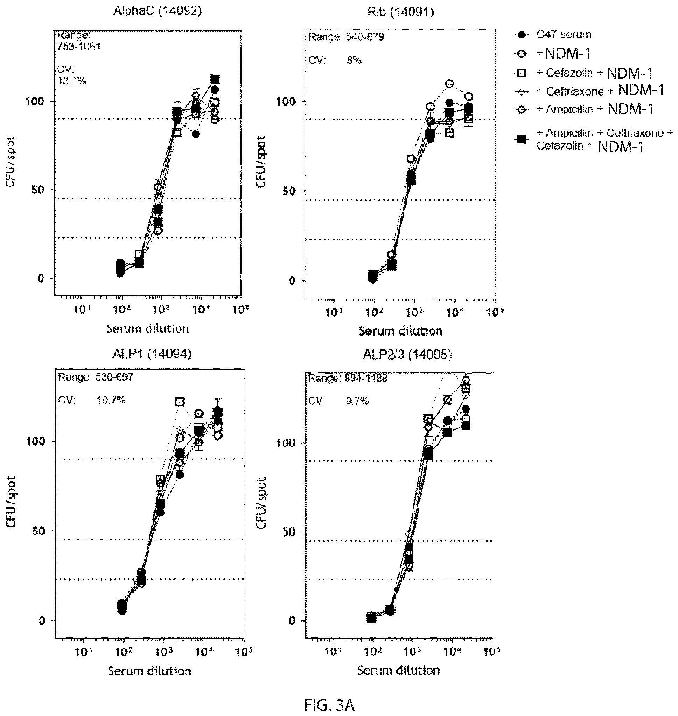

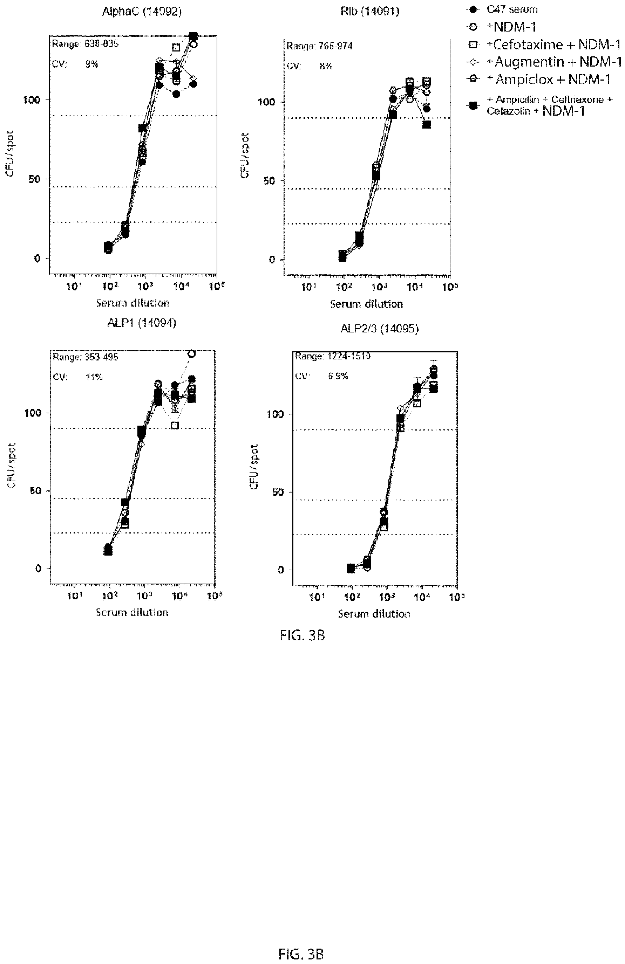

The results are shown in Fig. 3A and 3B which shows essentially identical OPkA killing curves for a test serum analyzed against all GASTON GBS strains in the absence or presence of high concentrations of beta-lactams and NDM-1 (0.2 µM). Specifically, the graphs in figure 3A and 3B show GBS bacterial survival (CFU) as a function of increasing serum dilution. For the example, one serum sample (identified as C47) was analyzed against the four indicated GASTON NCTC GBS strains with or without addition of NDM-1 (0.2 µM) only, or in combination with antibiotics. For each GASTON GBS strain, the samples indicated in the legend were analyzed within the same experiment and the titer range and intra-assay %CV achieved for serum ± indicated spiking conditions (NDM-1 ± indicated beta-lactam) are shown on each graph. Fig. 3A shows results for experiments assessing Cefazolin (839 µM), Ceftriaxone (604 µM), Ampicillin (1000 µM) and a beta-lactam cocktail consisting of Ampicillin, Ceftriaxone and Cefazolin (1000, 604 and 839 µM, respectively), whereas Figure 3B shows results for experiments assessing Cefotaxime (878 µM), Augmentin (200 µM based on clavulanic acid), Ampiclox (459 µM) and the same beta-lactam cocktail as in (A).

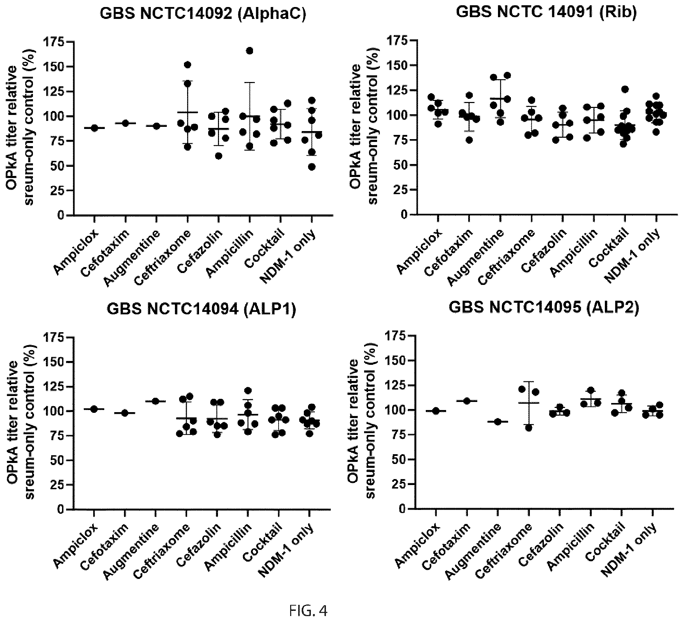

Figure 4 finally shows that shows that the result seen for a single serum in Fig 3A and 3B was reproduced when several sera were spiked with high concentrations of beta-lactams and tested in the OPkA against all four GBS target strains.

In total, six sera (n=6) were analyzed in the OPkA against indicated GBS strains with or without the addition of NDM-1 and indicated beta-lactams (NDM-1 added together with all beta-lactams). For Ceftriaxome, Cefazolin and Ampicillin, a full data set was generated for all GBS strains (n=6) except for NCTC14095 (Alp2), where three of the serum samples were tested so far (n=3). For Ampiclox, Cefotaxime and Augmentin a full data set was generated for NCTC14091 (Rib) (n=6) but only one serum (C47, shown in Fig 3A and 3B) was tested against NCTC14092 (AIpC), NCTC14094 (Alp1) and NCTC14095 (Alp2). Results show individual serum samples and are visualized as percentage of titer achieved for the same serum without addition of NDM-1 or beta-lactams.

## Claims

1. A method for preparing a sample for analysis, said method comprising:
a) providing a sample potentially containing an antimicrobial substance, such as an antibiotic; and
b) treating said sample with an enzyme to inactivate said antimicrobial substance.

2. The method according to claim 1, wherein said analysis comprises determining microbial viability, and preferably, wherein said analysis comprises determining immunological activity against microbes.

3. The method according to any one of the preceding claims, wherein said analysis is an Opsonophagocytosis killing Assay (OPkA assay).

4. The method to any one of the preceding claims, wherein said antimicrobial substance is a beta-lactam antibiotic.

5. The method according to claim 4, wherein said beta-lactam antibiotic is a penicillin, sporin, cephamycin, monobactam, carbapenem and/or carbacephem.

6. The method according to any one of the preceding claims, wherein said enzyme is a beta-lactamase, preferably a carbapenemase, more preferably a class B carbapenemase.

7. The method according to claim 6, wherein said class B carbapenemase is a New Delhi metallo-beta-lactamase (NDM).

8. The method according to claim 7, wherein said New Delhi metallo-beta-lactamase is New Delhi metallo-beta-lactamase 1 (NDM-1).

9. The method according to claim 8, wherein said NDM-1 comprises an amino acid sequence according to SEQ ID NO: 1, or a variant thereof having at least 95% sequence identity thereto.

10. The method according to any one of the preceding claims, wherein said sample is from a subject that has been vaccinated against a bacterial infection, or is the offspring of a subject that has been vaccinated against a bacterial infection.

11. The method according to claim 10, wherein said bacterial infection is a Group B streptococcal infection.

12. A method comprising a microbial viability assay of a sample, said method comprising the steps of:
a) preparing a sample for analysis according to the method of any one of claims 1-11; and
b) performing a microbial viability assay on the prepared sample of step a).

13. A New Delhi metallo-beta-lactamase 1 (NDM-1) polypeptide, said NDM-1 polypeptide comprising an amino acid sequence according to SEQ ID NO: 2, or a variant thereof having at least 95% sequence identity thereto.

14. A method for recombinantly producing a New Delhi metallo-beta-lactamase 1 (NDM-1) protein according to SEQ ID NO: 1 or a variant thereof having at least 95% sequence identity thereto, said method comprising the steps of:
a) expressing a polypeptide according to SEQ ID NO: 2, or a variant thereof having at least 95% sequence identity thereto, in a bacterial host cell, such as an *E. coli* host cell, and allowing said bacterial host to cleave off a PelB leader sequence from SEQ ID NO: 2, thereby providing an amino acid sequence according to SEQ ID NO: 3, or a variant thereof having at least 95% sequence identity thereto;
b) optionally lysing said bacterial host cell to release said amino acid sequence according to SEQ ID NO: 3, or said variant thereof, from the bacterial host cell;
c) using the His-tag of SEQ ID NO: 3, or the variant thereof, and affinity chromatography to obtain said amino acid sequence of SEQ ID NO: 3, or said variant thereof; and
d) using a TEV protease to cleave the His-tag off from the amino acid sequence of SEQ ID NO: 3, or said variant thereof, thereby rendering an NDM-1 protein according to SEQ ID NO: 1, or a variant thereof having at least 95% sequence identity thereto.

15. Use of an antimicrobial inactivating enzyme as defined in any one of claims 6-9 for preparing a sample for an analysis as defined in any one of claims 2-3.
